# EUROPEAN PATENT APPLICATION

(11) **EP 4 074 326 A1**
(43) Date of publication of application: **19.10.2022**
(21) Application number: 21168940.1
(22) Date of filing: 16.04.2021
(51) Int. Cl.: A61K 36/9068, A61K 31/05, A61P 3/00, A61K 31/12, A61P 35/00

(54) **COMBINATION PREPARATION CONTAINING RESVERATROL**

(71) Applicant: Löffler, Bernd-Michael, 10717 Berlin (DE)
(72) Inventor: LÖFFLER, Bernd-Michael., D10717 Berlin (DE); ADISSAR, Akhila, K., Thrissur 680309 Kerala (IN)
(74) Representative: Dr. Klemens Schubert Patentanwalt

(57) **Abstract**

Herein described is a combination preparation comprising resveratrol and at least one gingerol, or resveratrol and a combination of gingerols, in addition to pharmaceutical or nutraceutical acceptable adjuvants and additives

## Description

### Technical Field

The present invention relates to a combination preparation comprising resveratrol and at least one gingerol, in addition to pharmaceutical or nutraceutical acceptable adjuvants and additives.

### Background Art

Resveratrol, with the chemical name 3,5,4'-trihydroxy-*trans*-stilbene, is a type of a natural phenol and can be found in the skin of natural food like raspberries, blueberries, peanuts and grapes. It is known that resveratrol has antifungal and antibacterial properties which protect the plants from respective pathogens. Resveratrol is commonly used as dietary supplement. But unfortunately, until now no high-quality evidence could be detected for a substantial effect on human diseases. The reason of poor clinical efficacy of resveratrol in humans could be its poor bioavailability, fast metabolism and lack of biological stability, as well as the way of administration. The poor bioavailability can be due to the rapid conjugation of trans-resveratrol to glucuronic acid and sulfates, producing glucuronides and sulfate conjugates which accumulate in plasma and urine.

### Summary of Invention

One object of the present invention is a combination preparation comprising resveratrol and at least one gingerol, in addition to pharmaceutical or nutraceutical acceptable adjuvants and additives.

Preferred according to the invention is a combination preparation comprising resveratrol and a combination of gingerols.

Preferred according to the present invention is a combination preparation, wherein the gingerol or gingerols are selected from total ginger extract matrix. Especially preferred is herein, that the total ginger extract matrix is obtained from a supercritical carbon dioxide extraction.

Preferred is also a combination preparation, wherein the gingerols are selected from 6-gingerol, 8-gingerol, and 10-gingerol or a combination thereof. Especially preferred is herein, that the combination of the weight ratio of 6-gingerol to 8-gingerol to 10-gingerol is in the range from 0.1 to 1 to 10 up to 10 to 1 to 0.1.

Preferred is further a combination preparation, wherein the gingerols further comprise shogaols, which are selected from 4-shogaol, 6-shogaol, 8-shogaol, 10-shogaol, and 12-shogaol.

Further preferred is a combination preparation, wherein the combination preparation further comprises curcumin and/or curcuminoids, and wherein the curcuminoids are preferably selected from demethoxycurcumin or bisdemethoxycurcumin or their combination.

Especially preferred is a combination preparation, wherein the amount of the gingerol or the combination of gingerols, is in the range of 5 to 25 % (w/w), preferably in the range of 5 to 15 % (w/w), most preferably in the range of 7 to 13 % (w/w) in relation to resveratrol.

Preferred is also a combination preparation, additionally comprising cytochrome P450 inhibitors. Especially preferred herein are inhibitors selected from azole antimycotics, imidazole antimycotics, triazole antimycotics, macrolide antibiotics, HIV protease inhibitors, and the like.

One further object of the present invention is a combination preparation, according to the invention, in form of a microencapsulated product, comprising a homogenized and/or complexed mixture of the active compounds, namely resveratrol and gingerol or gingerols.

One further object of the present invention is a combination preparation, according to the invention, wherein the pharmaceutical composition is in form of an oral or parenteral or topical applicable formulation.

One further object of the invention is also a combination preparation, according to the invention, namely the combination of resveratrol and the unique gingerol complex, for treatment of cancer, heart diseases or diabetes, for prevention of insulin resistance, for lowering LDL cholesterol, for reduction of inflammation, for treatment of nausea, indigestion, flatulence, loss of appetite, heart burn, colic diarrhea, upset stomach, cough, headache and various infections, arthritic pain, low back ache and muscle soreness, for use as anti-cancer agent, for use as agent for neuroprotective, cardiovascular, respiratory, anti-obesity, antidiabetic, anti-nausea, antiemetic, antiviral, antioxidant diseases and for antimicrobial protection.

Another object of the invention is a method for the production of a combination preparation according to the invention, according to the following steps: extracting gingerols from ginger rhizomes by use of supercritical carbon dioxide extraction;
adding resveratrol to the extracted gingerols and mixing the compounds; adding agents for microencapsulation and homogenisation of the obtained mixture;
spray drying the obtained mixture and obtaining a powder, which is the final composition.

Another object of the invention is a combination preparation, according to the invention for use in the treatment of cancer, heart diseases or diabetes, for prevention of insulin resistance, for lowering LDL cholesterol, for reduction of inflammation, for treatment of nausea, indigestion, flatulence, loss of appetite, heart burn, colic diarrhea, upset stomach, cough, headache and various infections, arthritic pain, low back ache and muscle soreness, for use as anticancer agent, for use as agent for neuroprotective, cardiovascular, respiratory, anti-obesity, antidiabetic, anti-nausea, antiemetic, antiviral, antioxidant diseases and for antimicrobial protection.

Surprisingly it was found that the combination of resveratrol with at least one gingerol provides a synergistic effect in the medical activity of the combination preparation according to the invention.

The synergistic effect allows for a minor doses of the active ingredients and leads to a higher efficiency of the combination preparation according to the invention.

The object of the present invention is a bioavailable resveratrol formulation prepared by using synergistic microencapsulation process (SMP) technology and further using a unique gingerol complex. The object of the present invention is a bioavailable resveratrol formulation with gingerol complex. Resveratrol (3,5,4'-trihydroxy-trans-stilbene), is a part of a group of compounds called polyphenols, possessing two phenol rings linked to each other by an ethylene bridge. It is found in more than 70 plant species, especially in the skin and seeds of red grapes. For industrial purposes, resveratrol is generally obtained by chemical or biotechnological synthesis from yeast *Saccharomyces cerevisiae.* In plants, resveratrol acts as a phytoalexin that is synthesized in response to pathogens, mechanical injury and UV irradiation. The high concentrations of resveratrol in grapes leads to a response to fungal infection. Several studies have been done to prove the health benefits of resveratrol and they showed that resveratrol possesses an extremely high antioxidant potential. Its anti-ageing and disease fighting powers are also reported. Resveratrol also exhibits anti-tumor activity and has a great potential to prevent various types of cancers. It helps to prevent insulin resistance, to lower LDL cholesterol and to reduce inflammation. Thus, resveratrol makes the body healthy enough to resist the development of lifestyle disorders like heart disease and diabetes. From the research studies it is clear that resveratrol possess anticancer, cardioprotective, antimicrobial, antiaging, blood-sugar lowering, anti-inflammatory and neuroprotective properties. Experts say that even though it has enough potential, still there is not that much data to prove its effectiveness. Moreover, all the health benefits of resveratrol are hampered by its low bio availability. And due to rapid and extensive metabolism in liver, intestine and colon, the plasma level of conjugated metabolites are higher compared to the present amount of resveratrol. So there comes the need of a bioavailability enhancer for utilizing resveratrol as a supplement.

The promising therapeutic effects as well as lesser adverse effects give herbal extracts worldwide acceptance. But because of large molecular size and decreased lipid solubility, they are poorly absorbed from the intestine. Most of the plant products, including phenolics, are water soluble and that is the reason for their poor bioavailability, as they cannot cross the lipid membranes of the intestine. Many researches are performed on this area for developing advanced technologies for increasing their bioavailability. As a result, many new technologies have been found or developed to enhance absorption and hence bioavailability. But these new technologies also face some problems. For example, nanocrystallization helps in the delivery of poorly water-soluble drugs through different routes. But despite the benefits it offers, due to small size of nano crystals it can enter any cell of the body and that may lead to cytotoxicity. The preparation and stability of various drug nano crystals differ based on molecular structure. And this technology needs expensive equipment, which eventually increases the cost of the final product. However these new technological solutions using solvents as e.g. Tween20/80, synthetic surfactants etc. or nanotechnology, where the exact dose dependent toxicology is not established which may potentially decreasing the biological efficacy or increasing unwanted side effects not do to the herbal drug but to the delivery technology. Ayurveda has been utilizing various herbal drug combinations which include bioavailability enhancers of plant origin for centuries. Researches on those herbal extracts are on progress all over the world. Many bioavailability enhancers of herbal origin have been proved and ginger is one among them.

Ginger is one of the most widely used natural products since ancient times. Over 400 different compounds are found in ginger including polysaccharides, lipids, organic acids and raw fibers. The bioactive compounds in ginger are mainly phenolic and terpene compounds. The phenolic compounds are gingerols, shogaols, and paradols. In fresh ginger, gingerols are the major polyphenols, such as 6-gingerol, 8-gingerol, and 10-gingerol. Gingerol, a phyto-chemical phenol compound in fresh ginger, which is normally found as a pungent yellow oil, activates spice receptors on tongue. By cooking, gingerol is converted into zingerone, a comparatively less pungent form and with a spicy sweet aroma. When dried or mildly heated, gingerols become shogaols which are twice pungent of gingerols. Gingerol acts on the intestinal mucus membrane to facilitate absorption. It is found that gingerol causes vasodilation in gastro intestinal tract (GIT) and thus increases absorption of drugs. It also causes suppression of first pass metabolism and inhibition of drug metabolizing enzymes. Moreover, the compounds in ginger essential oil possess lipophilic properties. According to Ayurvedic principles, ginger possesses *theekshna guna* and *ushna veerya* (piercing and hot), which make it assimilate very fast in the body. So it is categorised under the category *deepaneeya gana* in Ayurveda treatings, which contain the drugs that improve digestive strength. The biologic activities of ginger include antioxidant, anti-inflammatory, antimicrobial, anticancer, neuroprotective, cardiovascular protective, respiratory protective, anti-obesity, antidiabetic, anti-nausea, and antiemetic activities. The phenolic compounds present in it are mainly responsible for these activities. As a medicine ginger is used for treating nausea, indigestion, flatulence, dysentery, loss of appetite, heart burn, colic, diarrhea, upset stomach, cough, headache and various infections. It is also good against arthritic pain, low back ache, muscle soreness. It is highly effective in joint problems because of its anti-inflammatory properties. Therapeutically it boosts circulation, lowers high blood pressure, and acts antiviral, in conditions like flu and cold. So, ginger can be considered as a safe, cost effective drug with high therapeutic potential and which is an active bioenhancer also.

The present invention is based on this traditional knowledge on ginger, the potential of this extract in increasing the blood circulation, bio availability enhancement of other bio active molecules. According to the invention, a unique ginger extract formulation in which there is more than one from of gingerol is present. The unique gingerol composition consist of unique ratio of 6-gingerol, 8-gingerol, 10-gingerol and the dehydrated gingerol which is shogaol.

The compounds of the combination preparation according to the invention can be prepared in different ways. Resveratrol is usually generated by a technical process of synthesis. It has to be pointed out that resveratrol may also be extracted from plants and fruits. Otherwise, the gingerols are extracted by a non solvented process, which is supercritical carbon dioxide-based extraction.

A further object of the present invention is the use of the resveratrol-gingerol combination in combination with curcumin or a curcuminoid preparation (comprising of CUR (curcumin), DMC (demethoxycurcumin) and BDMC (bisdemethoxycurcumin) as major components) specially for treatment of cancer, diabetes, and insulin resistance, either in one combined oral preparation or in separate oral preparations given at the same time.

### Description of Embodiments

### Definitions

The following definitions are given in order to explain the matter of the present invention clear and concise.

The term "resveratrol" describes 3,5,4'-trihydroxy-*trans*-stilbene as well as isomeric compounds and derivatives.

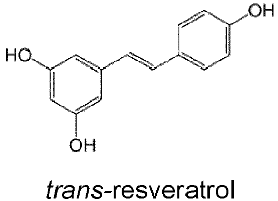

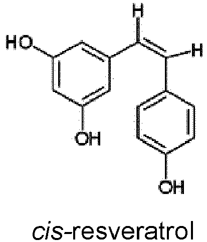

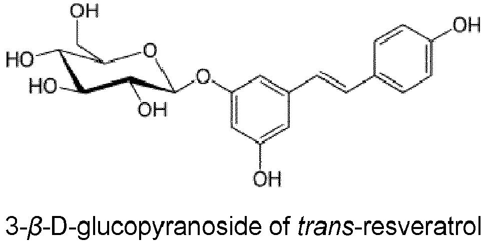

Resveratrol used according to the invention may be produced by chemical synthesis. Resveratrol may also be extracted from plants or fruits, which contain resveratrol and the respective derivates.

The terms "gingerol" or "gingerols" describes components of ginger plants, that are members of the ginger family, which are broadly used as dietary supplements or bioactive compounds for different diseases. Most important gingerols are 6-gingerol, 8-gingerol, 10-gingerol, and gingerols comprising shogaols, which are selected from 4-shogaol, 6-shogaol, 8-shogaol, 10-shogaol, 12-shogaol. The terms comprise also their tautomeric, isomeric or stereo-isomeric forms like keto-enol forms as well as other gingerol derivatives from the class of gingerols. The main compounds are shown in the following chemical formulas.

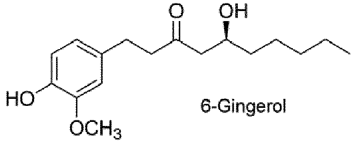

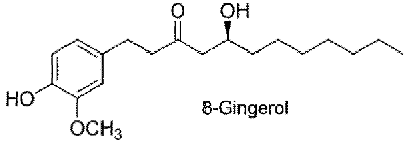

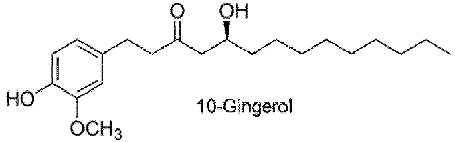

The terms "curcumin" or "curcuminoid" relate especially to the curcumin (CUR), demethoxycurcumin (DMC) and bisdemethoxycurcumin (BDMC), as well as to their isomeric forms, especially as keto-enol tautomeric forms, which are compounds present in curcuma plants.

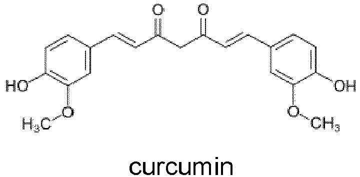

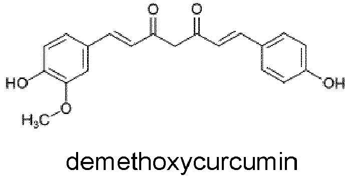

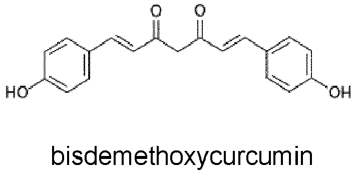

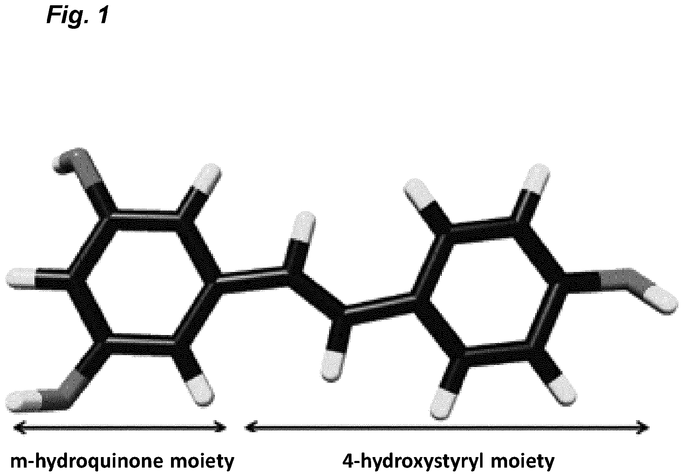

The term "resveratrol-gingerol complex" describes the combination of resveratrol and gingerol and/or gingerols and/ or curcumin and/or curcuminoids. Complex means that the compounds are combined in a manner to form stable complexes which have a high bioavailability and stability. This advantage is shown in Figure 1. Figure 1 shows that trans-resveratrol comprises a hydroquinone moiety and a hydroxystyryl moiety. These different moieties are susceptible to form complexes with gingerols by intra molecular hydrogen bonding (Van-der-Waals-Bond). This is the important factor to form stable complexes so that the biological stability of resveratrol has been improved. As evident from Ayurveda, the gingerols will improve the absorption of bioactive compounds, also improve the blood flow - because the gingerols make stable complexes with resveratrol. In that respect, the bioavailability of resveratrol is highly improved.

Therefore, the key in the present invention is the formation of a synergistic complex - microencapsulated system with resveratrol and gingerols.

### Pharmaceutical Compositions

The present invention is a stable, bio available, bio efficient, consistent delivery formulation of resveratrol analog along with unique gingerols complex, further micro encapsulated. The present invention uses an innovative, "synergistic microencapsulating process" (SMP) technology to complex and deliver resveratrol. This resveratrol-gingerol complex exhibits a very good thermal stability, even above 60°C. This product also having a very good bio availability as compared to the standard resveratrol in any other suspension/conventional delivery form.

In general, usual pharmaceutical or nutraceutical preparations can be produced according to the state of the art.

A preferred subject of the present invention is a pharmaceutical and/or nutraceutical composition for oral, buccal, sublingual, nasal, rectal, subcutaneous, intravenous or intramuscular application as well as for inhalation, which, in addition to the usual vehicle and dilution agents, also contains the active ingredients according to the present invention.

The pharmaceutical and/or nutraceutical compositions of the invention are produced, in the known way and in suitable dosage, with the usual solid or liquid vehicle substances or dilution agents and the usually used pharmaceutical-technical adjuvants corresponding to the desired type of application. The preferred compositions are present in a form of administration which is suitable for oral application. Such forms of administration include, for example, tablets, sucking tablets, film tablets, dragees, capsules, pills, powders, solutions, aerosols or suspensions or slow-release forms.

Corresponding tablets can be obtained, for example, by mixing the active substance with known adjuvants, for example, inert dilution agents such as dextrose, sugar, sorbitol, mannitol, polyvinylpyrrolidone, bursting agents such as corn starch or alginic acid, binders such as starches or gelantins, lubricants such as magnesium stearate or talc and/or agents for achieving a slow-release effect such as carboxypolymethylene, carboxymethylcellulose, cellulose acetate phthalate or polyvinyl acetate. The tablets may also consist of several layers.

Dragees can also be produced correspondingly, for controlled or delayed release forms of preparation, by coating the cores produced analogously to the tablets with agents commonly used in dragee coatings, for example, polyvinylpyrrolidone or shellac, gum arabic, talc, titanium dioxide or sugar. The dragee envelope may also consist of several layers, wherein the adjuvants mentioned above in the case of tablets can be used.

Solutions or suspensions containing the active substances used according to the invention may additionally contain agents that improve taste, such as saccharin, cyclamate or sugar, as well as, e.g., taste enhancers such as vanilla or orange extract. They may also contain suspension adjuvants such as sodium carboxymethylcellulose or preservatives such as p-hydroxybenzoate. Capsules containing active substances can be produced, for example, by mixing the active ingredients with an inert vehicle such as lactose or sorbitol and encapsulating this mixture in gelatin capsules. Suitable suppositories can be produced, for example, by mixing with vehicle agents provided therefore, such as neutral fats or polyethylene glycol or derivatives thereof.

The production of the pharmaceutical preparations or compositions according to the invention is known in and of itself, and is described in handbooks known to the person skilled in the art, for example, Hager's Handbuch [Handbook] (5th ed.) 2, 622-1045; List et al., Arzneiformenlehre [Instructions for Drug Forms], Stuttgart: Wiss. Verlagsges. 1985; Sucker et al., Pharmazeutische Technologie [Pharmaceutical Technology], Stuttgart: Thieme 1991; Ullmann's Enzyklopädie [Encyclopedia] (5th ed.) A 19, 241-271; Voigt, Pharmazeutische Technologie [Pharmaceutical Technology], Berlin: Ullstein Mosby 1995.

### Examples

The typical concentrations of resveratrol in the synergistic microencapsulated complex according to the present invention is 100 mg/dose, wherein the single oral dose is standardized, for example, to 500 mg in a vegetarian hard capsule.

### Example 1

| | |
|---|---|
| resveratrol | 100 mg |
| ginger extract (Batch C21-55) | 25 mg |
| modified food starch | 375 mg |
| *total amount* | 500 mg |

### Example 2

| | |
|---|---|
| resveratrol | 100 mg |
| 6-gingerol | 5 mg |
| 8-gingerol | 5 mg |
| 10-gingerol | 5 mg |
| modified food starch | 385 mg |
| *total amount* | *500 mg* |

### Example 3

| | |
|---|---|
| resveratrol | 100 mg |
| 6-gingerol | 15 mg |
| 8-gingerol | 3 mg |
| 10-gingerol | 2 mg |
| modified food starch | 380 mg |
| *total amount* | *500 mg* |

### Example 4

| | |
|---|---|
| resveratrol | 100 mg |
| ginger extract | 10 mg |
| 8-shogaol | 3 mg |
| 12-shogaol | 1 mg |
| modified food starch | 386 mg |
| *total amount* | *500 mg* |

### Example 5

| | |
|---|---|
| resveratrol | 100 mg |
| ginger extract | 25 mg |
| curcumin | 5 mg |
| demethoxycurcumin | 2 mg |
| modified food starch | 368 mg |
| *total amount* | *500 mg* |

### Example 6

| | |
|---|---|
| resveratrol | 100 mg |
| ginger extract | 25 mg |
| curcumin | 5 mg |
| bisdemethoxycurcumin | 1 mg |
| modified food starch | 369 mg |
| *total amount* | *500 mg* |

The combination preparations as described in the above-named examples are different for the use in different therapeutic issues. It is know in the state of the art that some of the used active ingredients like ginger extract, gingerols, shogaols, curcumin and curcuminoids provide different pharmaceutical actions. According to the disease or the combination of different diseases it is possible according to the invention to prepare specific combination preparations. This leads to an effective therapy.

### Example 7

A patient, suffering from a prostate carcinoma, was examined by checking a blood sample. Circulating human tumour cells (CTC's) characterised by Ep-CAM and additional cell surface markers have been used. The CTC's have been tested with different anti-cancer drugs. The tests are shown in Table 1. The resveratrol-gingerol preparation according to the invention as described in Example 1 showed a high reduction of the cancer cells in the blood sample. The combination preparation according to the invention showed in comparable concentrations a higher efficacy as curcuminoids. Furthermore, the positive effects could be shown to be dose dependent.

**Table 1**

| Concentration- and time-dependent specific in vitro vitality reduction (%) in the presence of eutherapeutic concentrations of | % |
|---|---|
| Oliphenolia | 75 |
| Curcumin (450 mg) | 80 |
| 25-OH-D3 (100ng/ml) | 25 |
| 25-OH-D3 (200ng/ml) | 30 |
| Methadone (3 x 15 drops) | 30 |
| CUREmin-ACTIF^{®} (3 x 3 capsules) | 70 |
| Artesunat | 60 |
| Resveratrol-gingerol-complex (Example 1) 1 dose | 90 |
| Resveratrol-gingerol-complex (Example 1) 10 doses | 99 |

### Manufacturing process

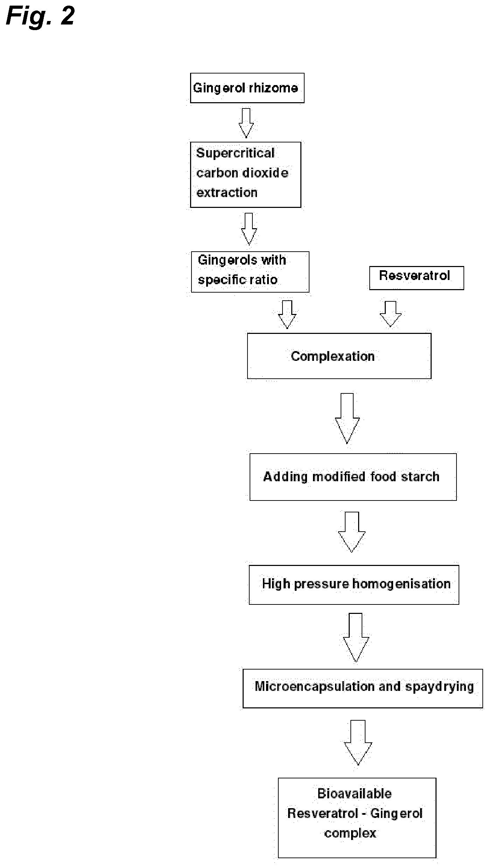

Figure 2 shows the process according to the present invention of the production of the combination preparation.

In a typical procedure, ginger rhizomes are extracted using supercritical carbon dioxide under mild temperatures. The resulting extract of gingerols are further purified using a resin matrix to optimize the ratio of gingerols. This extract thus obtained is used for the present formulation. Resveratrol and the ginger extract produced is mixed well and milled using a molecular miller to facilitate the complexation between resveratrol and gingerols. The functional groups in resveratrol and gingerols experience hydrogen bonding which is very important in forming a stable complex. The formation of the complex is evident by an FT - IR spectrograph. The complex thus produced is added to an aqueous slurry of modified food starch at a temperature of 40-45°C. This mixture is homogenized with a high pressure homogenizer. After homogenization, this colloidal solution is encapsulated using a spray drier with an inlet temperature of 175 -185°C and an out let temperature of 85-90°C. The resulting powder, which is the resveratrol-gingerols synergistic microencapsulated complex is characterized and encapsulated.

The microencapsulation process is performed under the use of modified food starch, which is selected from approved food additives.

The different combinations of the active ingredients are described in Examples 1 to 6, as shown above.
A great advantage of the present invention is that the combination of compounds from ginger rhizomes may be prepared in different processes. One process is to use the different compounds like gingerols, shogaols and curcuminoids and combine these compounds. A further process is to use a ginger extract received from ginger rhizome using supercritical carbon dioxide under mild temperatures. Still another process is to combine ginger extract with selected compounds like gingerols, shogaols and curcuminoids. Therefore, it is possible to combine selected active ingredients in order to use them for special therapeutical uses.

In Table 2 the composition of active ingredients extracted from ginger rhizomes is described. All batches described in Table 2 are produced from the same charge of ginger rhizomes. The extraction has been performed under different temperatures and the purification has been performed with different resin matrices. The results show that the compositions of the different substances present in the ginger rhizomes can be changed with the different steps in the extraction process. The main advantage is that the steps of combination of the active ingredients can also be performed with the production process and adding further active ingredients, available from other chemical or extraction processes from ginger rhizomes.

**Table 2**

| **Composition of gingerol extracts** | | | |
|---|---|---|---|
| **Batch No.** | **C20-11** | **C20-25** | **C21-55** |
| Temperature | -20°C | -10°C | 0°C |
| Resin matrix | Silica gel 60 | D-101 silica gel | Diaion^{®} HP-20 |
| Amount | wt% | wt% | wt% |
| 6-gingerol | 15.3 | 10.5 | 20.6 |
| 8-gingerol | 10.2 | 12.8 | 12.5 |
| 10-gingerol | 6.8 | 9.3 | 8.9 |
| 4-, 6-, 8-, 10-, 12-shogaol | 5.2 | 9.8 | 1.1 |
| Curcumin | 6.1 | 2.2 | 0.2 |
| Demethoxycurcumin | 1.1 | 0.9 | <0.01 |
| Bisdemethoxycurcumin | 0.5 | 0.2 | <0.01 |

## Claims

1. A combination preparation comprising resveratrol and at least one gingerol, in addition to pharmaceutical or nutraceutical acceptable adjuvants and additives.

2. The combination preparation, according to claim 1, comprising resveratrol and a combination of gingerols.

3. The combination preparation, according to claim 1 or claim 2, wherein the gingerol or gingerols are selected from total ginger extract matrix.

4. The combination preparation, according to claim 3, wherein the total ginger extract matrix is obtained from a supercritical carbon dioxide extraction.

5. The combination preparation, according to at least one of the preceding claims, wherein the gingerols are selected from 6-gingerol, 8-gingerol, and 10-gingerol or a combination thereof.

6. The combination preparation, according to claim 5, wherein the combination of the weight ratio of 6-gingerol to 8-gingerol to 10-gingerol is in the range of from 0.1 to 1 to 10 up to 10 to 1 to 0.1.

7. The combination preparation, according to at least one of the preceding claims, wherein the gingerols further comprise shogaols, which are selected from 4-shogaol, 6-shogaol, 8-shogaol, 10-shogaol, and 12-shogaol.

8. The combination preparation, according to at least one of the preceding claims, wherein the combination preparation further comprises curcumin and/or curcuminoids, and wherein the curcuminoids are preferably selected from demethoxycurcumin or bis bisdemethoxycurcumin or their combination.

9. The combination preparation, according to at least one of the preceding claims, wherein the amount of the gingerol or the combination of gingerols, is in the range of 5 to 25 % (w/w), preferably in the range of 5 to 15 % (w/w), most preferably in the range of 7 to 13 % (w/w) in relation to resveratrol.

10. The combination preparation, according to at least one of the preceding claims, additionally comprising cytochrome P450 inhibitors.

11. The combination preparation, according to at least one of the preceding claims, in form of a microencapsulated product, comprising a homogenized and/or complexed mixture of the active compounds, namely resveratrol and gingerol or gingerols.

12. The combination preparation, according to at least one of the preceding claims, wherein the pharmaceutical composition is in form of an oral or parenteral or topical applicable formulation.

13. The combination preparation, according to at least one of the preceding claims, for treatment of cancer, heart diseases or diabetes, for prevention of insulin resistance, for lowering LDL cholesterol, for reduction of inflammation, for treatment of nausea, indigestion, flatulence, loss of appetite, heart burn, colic diarrhea, upset stomach, cough, headache and various infections, arthritic pain, low back ache and muscle soreness, for use as anticancer agent, for use as agent for neuroprotective, cardiovascular, respiratory, anti-obesity, antidiabetic, anti-nausea, antiemetic, antiviral, antioxidant diseases and for antimicrobial protection.

14. Method for the production of a combination preparation according to at least one of the preceding claims, according to the following steps:
extracting gingerols from ginger rhizomes by use of supercritical carbon dioxide extraction;
adding resveratrol to the extracted gingerols and mixing the compounds;
adding agents for microencapsulation and homogenisation of the obtained mixture;
spray drying the obtained mixture and obtaining a powder, which is the final composition.

15. Combination preparation, according to at least one of the claims 1 to 13 for use in the treatment of cancer, heart diseases or diabetes, for prevention of insulin resistance, for lowering LDL cholesterol, for reduction of inflammation, for treatment of nausea, indigestion, flatulence, loss of appetite, heart burn, colic diarrhea, upset stomach, cough, headache and various infections, arthritic pain, low back ache and muscle soreness, for use as anticancer agent, for use as agent for neuroprotective, cardiovascular, respiratory, anti-obesity, antidiabetic, anti-nausea, antiemetic, antiviral, antioxidant diseases and for antimicrobial protection.
